Europäisches Patentamt

European Patent Office

Office européen des brevets

(19)

(11) Publication number: **0 210 791 B1**

# EUROPEAN PATENT SPECIFICATION

(12)

(45) Date of publication of patent specification: **13.11.91**

(51) Int. Cl.⁵: **C07D 487/18**, //(C07D487/18, 251:00,251:00,251:00)

(21) Application number: **86305508.3**

(22) Date of filing: **17.07.86**

The file contains technical information submitted after the application was filed and not included in this specification

(54) **Resotropin.**

(30) Priority: **31.07.85 US 760500**

(43) Date of publication of application:
**04.02.87 Bulletin 87/06**

(45) Publication of the grant of the patent:
**13.11.91 Bulletin 91/46**

(84) Designated Contracting States:
**AT BE CH DE FR GB IT LI LU NL SE**

(56) References cited:
**FR-A- 2 283 137**
**US-A- 2 414 416**

**THE CHEMISTRY OF HETEROCYCLIC COMPOUNDS, 1967, pages 583,584, Interscience Publishers Inc., New York, US; E.M. SMOLIN et al.: "s-Triazines and derivatives"**

**CHEMICAL ABSTRACTS, vol. 68, no. 15, 8th April 1968, page 6621, abstract no. 68699u, Columbus, Ohio, US**

(73) Proprietor: **INDSPEC CHEMICAL CORPORATION**
**Chamber of Commerce Building 411 Seventh Avenue**
**Pittsburgh Pennsylvania 15219(US)**

(72) Inventor: **Dressler, Hans**
**1236 Catalina Drive**
**Monroeville, PA 15146(US)**

(74) Representative: **Bull, Michael Alan et al**
**Haseltine Lake & Co. Hazlitt House 28 Southampton Buildings Chancery Lane**
**London WC2A 1AT(GB)**

## Description

This invention relates to the manufacture of resotropin.

Resotropin is a solid which can be formed by reaction of hexamethylenetetramine and resorcinol and which is useful, for example, in improving the adhesion of rubber to automobile tyre cords.

US-A-4,069,220 (Orem et al.) corresponding to FR-A-2283137 discloses a method of preparing resotropin in which the reaction of hexamethylenetetramine and resorcinol is carried out in an amount of solvent which is much less than that required to dissolve all of the reactants simultaneously. Water and several non-aqueous solvents are proposed for use in this method. Another method of preparing resotropin is disclosed, for example, in US-A-2,414,416 (Rhodes et al.).

C.A. 68 (1968), 68699u discloses a method of preparing resorcinol hexamethylenetetramine by reacting hexamethylenetetramine and resorcinol in water.

The Chemistry of Heterocylic Compounds : s-Triazines and derivatives, 1967, pages 583-584 discloses the reaction of hexamethylenetetramine with phenolic compounds.

It has now been discovered that white resotropin of much improved storage stability can be obtained in improved yields and purity by conducting the reaction of hexamethylenetetramine and resorcinol in methanol as solvent. The solvent is used in an amount sufficient to dissolve at least 50%, and preferably substantially all, of said reactants.

Thus, according to the present invention there is provided a method of preparing resotropin which comprises reacting hexamethylenetetramine and resorcinol in a solvent comprising an alcohol, characterised in that the solvent is methanol and in that the solvent is used in an amount sufficient to dissolve at least 50% of said reactants.

In the method of the invention, the hexamethylenetetramine and resorcinol are preferably each dissolved in a quantity of the solvent before being mixed together in a reactor.

The following Examples will serve to illustrate the invention. All parts and percentages in said Examples and elsewhere in the specification and claims are by weight unless otherwise specified.

### Example 1

(Comparison Example)

In accordance with the general procedure of Example 9 of US-A-4,069,220, a half-moon type agitator in a 1 l round bottom flask was charged with 154.0g (1.4m) resorcinol and mixed for five minutes with 196.2g (1.4m) hexamethylenetetramine. Then 24.4g of isopropanol was added during 15 minutes. The temperature of the mixture rose from 27° to 41°C and the mixture was cooled as an ice bath and agitated for 1.5 hours at 37° to 28°C. The mixture was lumpy and there was a buildup on the walls of the reactor. On discharge the lumps were broken up, vacuum-dried at 55°C, and analyzed by IR and UV spectroscopy, and for water solubility. The product contained a slight amount of water insolubles and the purity of resotropin was estimated at 93.8 percent (15 percent free hexamethylenetetramine by IR). The yield was 98 wt. percent.

### Example 2

To a stirred mixture, almost completely in solution, of 70.1g (0.5m) hexamethylenetetramine in methanol at 40°C contained in an apparatus similar to that used in Example 1 there was added in small portions, during 10 minutes, a solution of 55.0g (0.5m) resorcinol in 100ml methanol (no exotherm was observed). The mixture was agitated at about 40-45°C for 1.5 hr., then cooled to 25°C and suction-filtered onpaper in a Buchner funnel. The cake was pressed dry under a rubber dam, then air-dried overnight and vacuum-dried for 2 hours at 50-55°C/10mm Hg to give 107.0g (85.6 percent yield) of white solids. The purity was 99.6 percent.

### Example 3

(Recycle)

To an agitated mixture of 70.1g (0.5m) hexamethylenetetramine and mother liquor from Example 2 there was added a solution of 55.0g (0.5m) resorcinol in 25ml of mother liquor from Example 2 and 75ml make up methanol. The mixture was reacted and worked up as in Example 2. An off-white product (130.3g) was recovered for a yield of 104.2 percent and purity of 99.1 percent. The average yield of resotropin for Examples 2 and 3 was 94.9 percent and the average purity 99.4 percent.

Example 4

(Recycle)

An experiment was conducted by the general procedure of Example 2 but at twice the scale and a yield of 83.6 percent was obtained of white resotropin with a purity of 98.3 percent. The mother liquid was used in a subsequent run and resotropin was obtained of 97.4 percent purity (yield 101.2 percent). Mother liquid from the preceding run was used in a subsequent run and resotropin was obtained of 97.3 percent purity (yield 99.2 percent). The average yield and purity for Examples 2-4 was 94.7 percent (yield) and 98.7 percent (purity).

Example 5

(Comparison Example)

The general procedure of Exmaple 2 was repeated in aqueous medium; a solution of 660g (6.0m) resorcinol in 600ml of water was added to 840g (6.0m) hexamethylenetetramine in 2280ml water. The results obtained are compared in the following Table with the products prepared in accordance with Examples 2-4 above.

| Resotropin | Old, aqueous method (Example 5) | Method of the Invention (Examples 2-4) |
|---|---|---|
| Purity by %N | 95% | 97-99.6% |
| Colour | | |
| -fresh product | tan to grey | white to off-white |
| -after storage | dark brown (8 months) | white (14 months) |
| Yield, mole % | | |
| -once through | 70% (pilot plant)-81% (lab) | 84-86% |
| -with recycle | 95+% | 95+% |
| Solubility in water g/100g (after 1-2 months) | <1 | 10 |
| Water content,wt.% | 0.3 | 0.08 |
| Insolubles (water/ acetone) | 15-28 wt.% (after 1-7 months) | 0 (after 1 month) |
| TGA[a] wt.% loss (20°C/min, N$_2$ flow) | | |
| 1% at °C | 140 | 155 |
| 5% at °C | 165 | 175 |

a) TGA = Thermogravimetric analysis

The above Examples demonstrate the superiority of the method of the invention in making high-purity resotropin. While the product of Comparison Example 1 was lumpy, stuck to the reactor and had a purity of only 93.8 percent, the method of the invention produced purities as high as 99.6 percent and yields as high as 100 percent. Moreover, the product was more storage-stable.

Likewise inferior was the product of Example 5. It had a purity of only 95 percent, a yield of 81 percent (without recycle) and the product was not stable.

## Claims

1. A method of preparing resotropin which comprises reacting hexamethylenetetramine and resorcinol in a solvent comprising an alcohol, characterised in that the solvent is methanol and in that the solvent is used in an amount sufficient to dissolve at least 50% of said reactants.

2. A method according to claim 1, wherein methanol solutions of the reactants are reacted together.

3. A method according to claim 1 or 2, characterised in that sufficient solvent is employed to dissolve substantially all of the reactants.

4. A method according to claim 1, 2 or 3, characterised in that the resultant mother liquor is used for producing fresh resotropin.

**Revendications**

1. Procédé pour préparer la résotropine, qui comprend la réaction d'hexaméthylènetétramine et de résorcinol dans un solvant comportant un alcool, caractérisé en ce que le solvant est le méthanol et l'on utilise le solvant en une quantité suffisante pour dissoudre au moins 50% desdits réactifs.

2. Procédé selon la revendication 1, dans lequel on fait réagir ensemble des solutions méthanoliques des réactifs.

3. Procédé selon la revendication 1 ou 2, caractérisé en ce que l'on emploie suffisamment de solvant pour dissoudre pratiquement tous les réactifs.

4. Procédé selon la revendication 1, 2 ou 3, caractérisé en ce que l'on utilise la liqueur mère résultante pour préparer la résotropine fraîche.

**Patentansprüche**

1. Verfahren zur Herstellung von Resotropin, bei welchem Hexamethylentetramin und Resorzinol in einem alkoholhaltigen Lösungsmittel zur Reaktion gebracht werden, dadurch gekennzeichnet, daß als Lösungsmittel Methanol verwendet wird, und daß das Lösungsmittel in einer Menge verwendet wird, welche ausreicht, um mindestens 50 % der Reaktionspartner zu lösen.

2. Verfahren nach Anspruch 1, dadurch gekennzeichnet, daß Methanollösungen der Reaktionspartner zur Reaktion gebracht werden.

3. Verfahren nach Anspruch 1 oder 2, dadurch gekennzeichnet, daß das Lösungsmittel in einer ausreichenden Menge verwendet wird, um im wesentlichen den Gesamtgehalt der Reaktionspartner zu lösen.

4. Verfahren nach Anspruch 1, 2 oder 3, dadurch gekennzeichnet, daß die erhaltene Mutterlösung zur Herstellung von frischem Resotropin verwendet wird.